**Europäisches Patentamt**

(19) **European Patent Office**

**Office européen des brevets**

(11) Veröffentlichungsnummer: **0 206 143**

**B1**

(12) # EUROPÄISCHE PATENTSCHRIFT

(45) Veröffentlichungstag der Patentschrift:
**31.08.88**

(51) Int. Cl.⁴: **C 07 C 69/533**, C 07 C 67/333

(21) Anmeldenummer: **86108023.2**

(22) Anmeldetag: **12.06.86**

(54) **Verfahren zur Herstellung von 4-Pentensäureestern.**

(30) Priorität: **14.06.85 DE 3521381**

(43) Veröffentlichungstag der Anmeldung:
**30.12.86 Patentblatt 86/52**

(45) Bekanntmachung des Hinweises auf die Patenterteilung:
**31.08.88 Patentblatt 88/35**

(84) Benannte Vertragsstaaten:
**BE CH DE FR GB IT LI NL**

(56) Entgegenhaltungen:
**EP-A-0 126 349**
**DE-A-3 040 432**

(73) Patentinhaber: **BASF Aktiengesellschaft, Carl-Bosch- Strasse 38, D-6700 Ludwigshafen (DE)**

(72) Erfinder: **Hoelderich, Wolfgang, Dr., Mannheimer Strasse 18 c, D-6710 Frankenthal (DE)**
Erfinder: **Richter, Wolfgang, Dr., Am Huettenwingert 16, D-6706 Wachenheim (DE)**
Erfinder: **Lendle, Hubert, Dr., Ruedigerstrasse 41, D-6700 Ludwigshafen (DE)**
Erfinder: **Malsch, Klaus- Dieter, Dr., Amselweg 14, D-6707 Schifferstadt (DE)**

**Beschreibung**

Bei der Herstellung von Pentensäureestern durch Umsetzung von Butadien mit Kohlenmonoxid und Alkoholen in Gegenwart von Metallcarbonylkatalysatoren, wie es zum Beispiel in der DE-OS-3 040 432 beschrieben wird, erhält man erhebliche Mengen an isomeren Pentensäureestern. Für weitere Umsetzungen, z.B. zum Herstellen von δ-Formylvaleriansäureestern durch Hydroformylierung von Pentensäureestern wird jedoch 4-Pentensäureester als Ausgangsverbindung bevorzugt. Es wurde deshalb schon versucht, 4-Pentensäureester durch Isomerisierung von isomeren Pentensäureestern zu erhalten. Wie aus Bull. of the Chem. Soc. of Japan, Band 46, Seite 528 bekannt ist, erhält man durch Isomerisieren von 3-Pentensäuremethylester in Gegenwart von Cobaltcarbonylen jedoch vorwiegend 2-Pentensäuremethylester. Nach einer anderen in Tetrahedron Vol. 28, Seiten 5769-77 (1972) beschriebenen Arbeitsweise gelingt es zwar in Gegenwart eines Komplexes aus Rhodiumtriphenylphosphin und Zinnchlorid das Isomerengleichgewicht so zu verschieben, daß 4-Pentensäureester erhalten werden. Der dort verwendete Katalysator wird jedoch innerhalb weniger Stunden inaktiv.

In der EP-Anmeldung 126 349 wird ein Verfahren zur Herstellung von 4-Pentensäureester aus einem Pentensäureestergemisch durch Behandeln mit sauren Zeolithen, die einen Gehalt an Palladium, Ruthenium oder Rhodium haben, beschrieben.

Bei der Isomerisierung von Pentensäureestern liegen nach Erreichen des thermodynamischen Gleichgewichts fünf Isomere, nämlich 4-Pentensäureester, cis- und trans-3-Pentensäureester sowie cis- und trans-2-Pentensäureester vor, wobei das Gleichgewicht stark nach der Seite des trans-2-Pentensäureesters verschoben ist.

Es war deshalb die technische Aufgabe gestellt, ein Verfahren zur Herstellung von 4-Pentensäureestern aus isomeren Pentensäureestern zur Verfügung zu stellen bei dem das verwendete Katalysator eine lange Lebensdauer hat und leicht regenerierbar ist und zudem die lineare Verschiebung der Doppelbindung zum 4-Pentensäureester bevorzugt verläuft und zudem möglichst wenig des schwer abtrennbaren cis-2-Pentensäureesters gebildet wird.

Diese technische Aufgabe wird gelöst in einem Verfahren zur Herstellung von 4-Pentensäureestern durch Isomerisierung, wobei man isomere Pentensäureester bei erhöhter Temperatur mit Zeolithen behandelt und 4-Pentensäureester aus dem Reaktionsgemisch abdestilliert, dadurch gekennzeichnet, daß man Zeolithe des Pentasiltyps verwendet.

Das neue Verfahren hat den Vorteil, daß durch die verwendeten Pentasil-Zeolithe, die Doppelbindung vorzugsweise unter Bildung von 4-Pentensäureestern verschoben und die Bildung von cis-2-Pentensäureestern nahezu vollständig unterdrückt wird. Weiter hat das neue Verfahren den Vorteil, daß die verwendeten Pentasil-Zeolithe eine längere Lebensdauer haben und ohne Aktivitätsverlust leichter regenerierbar sind. Ferner hat das neue Verfahren den Vorteil, daß die verwendeten Pentasil-Zeolithe bereits ohne Zusatz von katalytisch aktiven Metallen die lineare Verschiebung der Doppelbindung zu 4-Pentensäureestern katalysieren. Zudem ist das anfallende Reaktionsgemisch leichter durch Destillation trennbar.

Vorteilhaft geht man von isomeren Pentensäureestern, z. B. 2- oder 3-Pentensäureestern aus, die sich von Alkoholen mit bis zu 12 Kohlenstoffatomen herleiten. Besonders bevorzugt verwendet man isomere Pentensäurealkylester, insbesondere von Alkoholen mit bis zu 4 Kohlenstoffatomen. Geeignete Ausgangsstoffe sind beispielsweise 3-Pentensäuremethylester, 3-Pentensäureethylester, 3-Pentensäurepropylester, 3-Pentensäurebutylester oder 2-Pentensäuremethylester, 2-Pentensäureethylester sowie 2-Pentensäurepropylester. Geeignet sind auch Gemische aus isomeren Pentensäureestern, wie sie bei der Umsetzung von Butadien mit Kohlenmonoxid und Alkoholen in Gegenwart von Metallcarbonylen entsprechend der DE-OS-3 040 432 erhalten werden. Besonders bevorzugt sind 3-Pentensäureester.

Die Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden ind. Das Verhältnis der Silicium- und Aluminiumatome zu Sauerstoff beträgt 1 : 2. Die Elektrovalenz der Aluminium enthaltenden Tetraeder ist durch Einschluß von Kationen in dem Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

Entsprechend ihrer Struktur werden Zeolithe in Verschiedene Gruppen unterteilt (vgl. Ullmanns Ecyclopädie d. techn. Chemie, 4. Aufl., Band. 24, Seite 575 (1983)). So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z. B. in Form eines Kuboktaeders, der aus Vierringen bzw. Sechsringen aufgebaut ist. Je nach Verknüpfung der Kuboktaeder, wodurch unterschiedlich große Hohlräume und Poren entstehen, unterscheidet man in Zeolithe vom Typ A, X oder Y.

Nach dem erfindungsgemäßen Verfahren werden Zeolithe des Pentasiltyps als Katalysatoren verwendet, die als Grundbaustein einen aus $SiO_4$-Tetraedern aufgebauten Fünfring gemeinsam haben. Sie sind durch ein hohes Verhältnis von $SiO_2$ zu $Al_2O_3$ gekennzeichnet, sowie durch Porengrößen, die zwischen denen der Zeolithe vom Typ A und denen vom Typ X oder Y liegen (vgl. Ullmanns Encyclopädie d. techn. Chemie, 4. Aufl., Bd. 24, 1983).

Die verwendeten Zeolithe können unterschiedliche Zusammensetzung aufweisen. Es handelt sich hierbei um Alumino-, Boro-, Eisen-, Gallium-, Chrom-, Arsen- und Wismutsilikatzeolithe oder deren Gemische sowie um Alumino-, Boro-, Gallium- und Eisengermanatzeolithe oder deren Gemische. Insbesondere sind die

2

isotaktischen Zeolithe, wie sie in der DE-OS-3 006 471 beschrieben sind, zu nennen.

Besonders eignen sich die Alumino-, Boro- und Eisensilikatzeolithe des Pentasiltpys für die Isomerisierung der Pentensäureester. Der Aluminosilikatzeolith wird z. B. aus einer Aluminiumverbindung, vorzugsweise Aluminiumhydroxid oder Aluminiumsulfat und einer Siliciumkomponente vorzugsweise hochdispersem Siliciumdioxid in wäßriger Aminlösung, insbesondere in 1,6-Hexandidmin-, 1,3-Propandiamin- oder Triethyletramin-Lösung mit oder ohne Alkali- oder Erdalkali-Zusatz bei 100 bis 220°C unter autogenem Druck hergestellt. Die erhaltenen Aluminosilikat-Zeolithe enthalten je nach Wahl der Einsatzstoffmengen ein Verhältnis von $SiO_2$ zu $Al_2O_3$ von 10 bis 40.000, insbesondere 30 bis 1000. Derartige Aluminosilikat-Zeolithe lassen sich auch in etherischem Medium wie Diethylenglykoldimethylether in alkoholischem Medium wie Methanol 1,4-Butandiol oder in Wasser synthetisieren.

Der Borosilikatzeolith wird z. B. bei 90 bis 200°C unter autogenem Druck synthetisiert, indem man eine Borverbindung wie Borsäure mit einer Siliciumverbindung, vorzugsweise hochdispersem Siliciumdioxid in wäßriger Aminlösung, insbesondere in 1,6-Hexandiamin-, 1,3-Propandiamin- oder Triethylentetramin-Lösung mit oder ohne Alkali- oder Erdalkalizusatz umsetzt. Solche Borosilikatzeolithe sind auch erhältlich, wenn man die Reaktion statt in wäßriger Aminlösung in etherischer Lösung, z. B. Diethylenglykoldimethylether oder in alkoholischer Lösung, z. B. 1,6-Hexandiol durchführt.

Den Eisensilikatzeolith erhält man z. B. aus einer Eisenverbindung, vorzugsweise Eisen(III)sulfat und einer Siliciumverbindung, vorzugsweise hochdispersem Siliciumdioxid in wäßriger Aminlösung, insbesondere 1,6-Hexandiamin, mit oder ohne Erdalkalizusatz bei einer Temperatur von 100 bis 200°C unter autogenem Druck.

Die so hergestellten Alumino-, Boro- oder Eisensilikatzeolithe werden nach ihrer Isolierung in der Regel bei 100 bis 160°C, vorzugsweise 110 bis 130°C getrocknet, dann bei 450 bis 550°C, insbesondere 490 bis 530°C calciniert und mit einem Bindemittel im Gewichtsverhältnis 90 : 10 bis 40 : 60 zu Strängen oder Tabletten verformt. Als Bindemittel eignen sich Aluminiumoxide, vorzugsweise Boehmit, hochdisperses Aluminiumoxid, amorphe Aluminosilikate mit einem Verhältnis von $SiO_2$ zu $Al_2O_3$ von 25 : 75 bis 90 : 10, vorzugsweise 75 : 25 Siliciumdioxid, bevorzugt hochdisperses Siliciumdioxid, Gemische aus hochdispersem Siliciumdioxid und hochdispersem Aluminiumoxid, hochdisperses Titandioxid sowie Ton. Nach der Verformung, werden die Extrudate oder Preßlinge vorteilhaft bei 100 bis 160°C für einen Zeitraum bis zu 16 Stunden getrocknet und anschließend bei 450 bis 550°C für einen Zeitraum bis zu 24 Stunden calciniert.

Man erhält auch vorteilhafte Katalysatoren, wenn die isolierten Alumino-, Boro- oder Eisensilikatzeolithe direkt nach der Trocknung verformt und erstmals nach der Verformung calciniert werden. Die erfindungsgemäßen Alumino-, Boro- oder Eisensilikatzeolithe können aber auch in reiner Form, ohne Binder als Stränge oder Tabletten verwendet werden. Die Verformung erfolgt dann unter Zusatz von Verstrangungshilfsmitteln oder Peptisierungsmitteln wie Hexaethylcellulose, Stearinsäure, Kartoffelstärke, Ameisensäure, Essigsäure, Oxalsäure, Salpetersäure, Ammoniak, Aminen, Silicoestern, Graphit oder deren Gemische.

Liegen die Zeolithe aufgrund der Art ihrer Herstellung nicht in der bevorzugten aciden H-Form vor, sondern z. B. in der Na-Form, dann kann diese vorteilhaft durch Ionenaustausch mit Ammoniumionen und anschließende Calcinierung oder durch Behandlung mit Säuren vollkommen oder partiell in die H-Form übergeführt werden.

Falls bei der erfindungsgemäßen Verwendung des zeolithischen Katalysatoren eine durch Koksabscheidung bedingte Desaktivierung eintritt, empfiehlt es sich, die Zeolithe durch Abbrennen der Koksablagerung mit Luft oder mit einem Gemisch aus Luft und Stickstoff, bei 400 bis 550°C, vorzugsweise 490 bis 550°C zu regenerieren. Die Zeolithe erhalten dadurch ihre Anfangsaktivität zurück.

Vorteilhaft haben die erfindungsgemäß verwendeten Zeolithe einen Gehalt an Alkalimetall-Ionen wie Natriumionen und/oder Erdalkali-Ionen wie Calcium oder Magnesium. Der Gehalt an Alkali- und/oder Erdalkali-Ionen kann einen Zeolithen bereits bei der Synthese durch entsprechende Wahl der Ausgangsstoffe vorgesehen werden oder an den fertigen Zeolithen durch Ionenaustausch oder durch Imprägnierung mit den entsprechenden Metallsalzen erzielt werden. Der Gehalt an Alklaimetall- und/oder Erdalkalimetallionen beträgt vorteilhaft von 0,05 bis 3,5 Gew.-%, bezogen auf Zeolith. ·

Es hat sich ferner bewährt, wenn die erfindungsgemäß verwendeten Zeolithe des Pentasiltyps einen Gehalt an Metallen mit der VIII. Gruppe des Periodensystems haben. Bevorzugte Metalle sind beispielsweise Kobalt, Eisen, Nickel, ferner Edelmetalle wie Palladium, Platin, Rhodium, Ruthenium oder Osmium. Vorzugsweise beträgt der Gehalt an den vorgenannten Metallen von 0,1 bis 7,0 Gew.-%, bevorzugt 0,5 bis 3,5 Gew.-%, bezogen auf Zeolith. Zweckmäßig bringt man die Übergangsmetalle in den verwendeten Zeolith ein, indem man den verformten Zeolithen in einem Steigrohr vorlegt und bei 20 bis 100°C eine wäßrige und/oder aminhaltige Lösung eines Halogenids oder Nitrats der genannten Metalle überleitet. Ein derartiger Ionenaustausch wird z. B. an der Wasserstoff-, Ammonium- und Alkaliform des Zeoliths vorgenommen. Eine weitere Möglichkeit der Metallaufbringung auf den Zeolithen besteht darin, indem man das zeolithische Material z. B. mit einem Halogenid, einem Nitrat oder einem Oxid der Übergangsmetalle in wäßriger und/oder alkoholischer und/oder aminhaltiger Lösung imprägniert. Sowohl an einem Ionenaustausch als auch an eine Imprägnierung schließt sich zumindestens eine Trocknung und wahlweise auch eine abermalige Calcinierung an.

Ferner ist es vorteilhaft, wenn die Katalysatoren einen Gehalt z. B. von 0,1 bis 4 Gew.-%, bevorzugt 0,5 bis 2,5 Gew.-% an seltenen Erdmetallen, insbesondere Cer und/oder Lanthan haben.

Eine mögliche Ausführungsform besteht z. B, darin, daß man Cobaltnitrat oder Cernitrat oder Lanthannitrat in Wasser löst und mit dieser Lösung den verformten oder unverformten Zeolith für eine geraume Zeit, in der Regel bis zu 30 Minuten tränkt. Anschließend wird das überschüssige Wasser am Rotationsverdampfer entfernt. Danach wird der getränkte Zeolith bei 100 bis 160°C getrocknet und bei 450 bis 550°C calciniert. Dieser Tränkvorgang kann mehrmals hintereinander vorgenommen werden, bis der gewünschte Metallgehalt erreicht ist.

Nach einer anderen Arbeitsweise wird der reine pulverförmige Zeolith in einer wäßrigen Cobalt-Nitrat-Lösung oder ammoniakalischen Palladium-Nitrat-Lösung aufgeschlämmt und für einen Zeitraum von 2 bis 24 Stunden bei 40 bis 100°C behandelt. Nach dem Abfiltrieren und Trocknen bei 100 bis 160°C und Calcinieren bei 450 bis 550°C wird der so dotierte Zeolith mit oder ohne Bindemittel zu Strängen oder Peletts weiter verarbeitet.

Ein Ionenaustausch des in der H-Form oder Ammonium-Form vorliegenden Zeolithen kann z. B. auch so vorgenommen werden, daß man den mit und ohne Bindemittel verformten Zeolithen in Strängen oder Pellets in einer Kolonne vorlegt und darüber z. B. eine wäßrige Cernitrat-Lösung oder Kobaltnitrat-Lösung bei einer Temperatur von 30 bis 80°C und für 15 bis 20 Stunden im Kreislauf leitet. Anschließend wird mit Wasser ausgewaschen, getrocknet und calciniert. Ein solcher Ionenaustausch kann auch mit ammoniakalischer Palladiumnitrat-Lösung durchgeführt werden.

Es hat sich bewährt, Zeolithe mit einem Metallgehalt, insbesondere Edelmetallgehalt bei einer Temperatur von 200 bis 550°C mit Wasserstoff z. B. für 1 bis 5 Stunden zu behandeln. Vorteilhaft wird vor der Behandlung mit Wasserstoff der Katalysator langsam in Gegenwart von molekularem Sauerstoff oder solchen enthaltenden Gasen auf eine Temperatur von bis zu 300°C erhitzt. Zudem ist es vorteilhaft, wenn der verwendete Wasserstoff einen Gehalt an Olefinen mit 2 bis 4 Kohlenstoffatomen hat.

Eire weitere Möglichkeit der Modifizierung besteht darin, daß man den Zeolithen - verformt oder unverformt - mit Säuren wie Salzsäure, Flußsäure, Phosphorsäure und/oder mit Wasserdampf behandelt. Darüber hinaus ist es möglich durch partielle Verkokung die Aktivität des Katalysators für ein Selektivitätsoptimum des gewünschten Reaktionsprodukts einzustellen.

Die erfindungsgemäßen Zeolithe vom Pentasilittyp können wahlweise als Stränge von 2 bis 4 mm oder als Tabletten mit einem Durchmesser von 3 bis 5 mm oder als Pulver mit einer Teilchengröße von 0,1 bis 0,5 mm oder als Wirbelgut verwendet werden.

Die Isomerisierung führt man vorteilhaft bei einer Temperatur von 50 bis 300°C durch. Hierbei hält man in der Regel Normaldruck, Unterdruck oder auch erhöhten Druck, z. B. bis zu 50 bar ein.

In einer diskontinuierlichen Arbeitsweise wird beispielsweise in einem Rührbehälter pulverförmiger Zeolith im isomeren Pentensäureestergemisch suspendiert und bei einer Temperatur von 50 bis 250°C, insbesondere 100 bis 200°C für einen Zeitraum von 2 bis 20 Stunden gerührt. Anschließend wird nach Abtrennen des Katalysators 4-Pentensäuremethylester durch Destillation isoliert.

In einer bevorzugten, kontinuierlichen Arbeitsweise wird in einem Reaktionsrohr verformter Zeolith des Pentasiltyps vorgelegt und die zu isomerisierende Mischung aus isomeren Pentenestern mit einer mittleren Verweilzeit von 5 bis 100 Minuten bei einer Temperatur von 50 bis 250°C, insbesondere 100 bis 200°C, vorzugsweise 100 bis 150°C flüssig durch das Rohr gepumpt. Anschließend wird das Reaktionsgemisch nach üblichen Methoden, z. B. destillativ getrennt und nicht umgesetzte Ausgangsstoffe vorteilhaft wieder zurückgeführt.

In einer besonders bevorzugten Ausführungsform des erfindungsgemäßen Verfahrens leitet man die isomeren Pentensäureester gasförmig gegebenenfalls zusammen mit einem inerten Trägergas wie Stickstoff über die erfindungsgemäßen Zeolithe des Pentasiltyps. Hierbei hält man vorteilhaft eine Temperatur von 150 bis 300°C, insbesondere 150 bis 250°C ein. Es hat sich bewährt, eine Belastung WHSV (Gramm Einsatzgemisch je Gramm Katalysator und Stunde) von 0,1 bis 20 h⁻¹, insbesondere 0,5 bis 5 h⁻¹ einzuhalten. Das erhaltene gasförmige Reaktionsgemisch wird kondensiert und 4-Pentensäuremethylester durch Destillation gewonnen.

Nach dem erfindungsgemäßen Verfahren erhältliche 4-Pentensäureester eignen sich zur Herstellung von δ-Formylvaleriansäureestern, einem Vorprodukt für die Herstellung von ε-Caprolactam, Hexandiol oder Adipinsäure.

Das Verfahren sei an folgenden Beispielen veranschaulicht:

**Katalysator A**

Ein Aluminosilikatzeolith vom Pentasil-Typ wurde unter hydrothermalen Bedingungen bei autogenem Druck und 150°C aus 650 g hochdispersem $SiO_2$, 203 g $Al_2(SO_4)_3$, 18 $H_2O$ in 10 kg einer wäßrigen 1,6-Hexandiamin-Lösung (Mischung 50 : 50 Gew.-%) in einem Rührautoklaven hergestellt. Nach Abfiltrieren und Auswaschen wurde das kristalline Reaktionsprodukt bei 110°C/24 h getrocknet und bei 500°C calciniert. Dieser Aluminosilikatzeolith enthielt 92,8 Gew.-% $SiO_2$ und 4,2 Gew.-% $Al_2O_3$.

Katalysator A wird erhalten durch Verformung dieses Aluminosilikatzeolithen mit Boehmit (Gewichtsverhältnis 60 : 40) zu 2 mm-Strängen, die bei 100°C/16 h getrocknet und bei 500°C/16 h calciniert wurden.

## Katalysator B

Der Zeolith wurde in einer hydrothermalen Synthese aus 640 g $SiO_2$ (hochdisperse Kieselsäure), 122 g $H_3BO_3$, 8000 g einer wäßrigen Hexandiamin-Lösung (Mischung 50 : 50 Gew.-%) bei 170°C unter autogenem Druck in einem Rührautoklaven hergestellt. Nach Abfiltrieren und Auswaschen wurde das kristalline Reaktionsprodukt bei 100°C/24 h getrocknet und bei 500°C/24 h calciniert. Es wurde ein Borosilikatzeolith vom Pentasiltyp erhalten, der 94,2 Gew.-% $SiO_2$ und 2,3 Gew.-% $B_2O_3$ enthielt.

Katalysator B wird erhalten durch Verformung dieses Borosilikatzeolithen mit Boehmit (Gewichtsverhältnis 60 : 40) zu 2 mm-Strängen, die bei 110°C/16 h getrocknet und bei 500°C/16 h calciniert wurden.

## Katalysator C

Katalysator C wird wie Katalysator B hergestellt, nur verwendet man als Verstrangungsmitten hochdisperses $Al_2O_3$ statt Boehmit.

## Katalysator D

Durch Behandlung des Katalysators B mit 20-%-iger $NH_4Cl$-Lösung im Gewichtsverhältnis 1 : 15 bei 80°C/2 h und durch anschließenden Ionenaustausch mit einer ammoniakalischen $Pd(NH_3)_4(NO_3)_2$-Lösung bei 50°C erhält man Katalysator D, der nach Trocknung bei 100°C und Calcination bei 500°C/5 h eine Pd-Gehalt von 0,8 Gew.-% aufweist.

## Katalysator E

Katalysator E wird hergestellt wie Katalysator D, besitzt aber einen Pd-Gehalt von 3,3 Gew.-%.

## Katalysator F

Katalysator F wird erhalten, indem man das Borosilikatzeolith-Pulver - beschrieben bei Katalysator B - ohne Zusatz von Bindemitteln zu 2 mm-Strängen verstrangt, bei 100°C trocknet und bei 500°C/16 h calciniert, danach diese Stränge mit 20-%-iger $NH_4Cl$-Lösung im Gewichtsverhältnis 1 : 15 bei 80°C/2 h behandelt und diese behandelten Stränge mit einer ammoniakalischen $Pd(NH_3)_4(NO_3)_2$-Lösung bei 5°C ionenaustauscht danach bei 110°C trocknet und bei 5500°C/5 h calciniert. Katalysator F enthält 2,1 Gew.-% Pd.

## Katalysator G (Vergleich)

Es wird als Katalysator ein Y Zeolith, der mit 0,5 Gew.-% Palladium beladen ist, gemäß EP-A-126 349 verwendet.

## Isomerisierung

Die Versuchsergebnisse, die mit den oben beschriebenen Katalysatoren erzielt werden, sind in Tabelle 1 zusammengestellt. Als Einsatzstoff wurde der 3-Pentensäuremethylester gewählt.

Die Reaktionen wurden in der Gasphase unter isothermen Bedingungen in einem Rohrreaktor (Werdel, 0,6 cm Innendurchmesser 90 cm Länge) mindestens 6 Stunden lang durchgeführt. Die Reaktionsprodukte wurden nach üblichen Methoden abgetrennt und charakterisiert. Die quantitative Bestimmung der Reaktionsprodukte und der Ausgangsprodukte erfolgte gaschromatographisch.

## Beispiel 8

In einem Reaktor mit 80 g Katalysatorfüllung werden 30 ml 3-Pentensäuremethylester pro Stunde bei 180°C h⁻¹ an Katalysator E über eine Laufzeit von 120 h umgesetzt. Der anfängliche 4-Pentensäureesteranteil im Produktgemisch von 8,8 Gew.-% fällt während dieser Zeit auf 6,5 Gew.-% ab.

## Vergleichsbeispiel 2

Man verfährt wie im Beispiel 8, verwendet jedoch den Katalysator G. Unter gleichen Reaktionsbedingungen fällt der Anteil an 4-PSE von 10,7 Gew.-% bereits nach 6 Stunden auf 6,7 Gew.-%.

## Tabelle 1

Isomerisierung von 3-Pentensäuremethylester (3-PSE)

| Beispiel | 1 | 2 | 3 | 4 | 5 | 6 | 7 | Vergl. 1 |
|---|---|---|---|---|---|---|---|---|
| Katalysator | A | B | C | D | E | E | F | G |
| Temperatur | 180°C | 180°C | 180°C | 180°C | 180°C | 180°C | 180°C | 180°C |
| WHSV | 2 h⁻¹ | 1 h⁻¹ | 1 h⁻¹ | 1,4 h⁻¹ | 1 h⁻¹ | 2 h⁻¹ | 2,5 h⁻¹ | 2 h⁻¹ |
| **Produktzusammensetzung Gew.-%** | | | | | | | | |
| 4-PSE | 3,5 | 3,7 | 5,1 | 8,0 | 9,8 | 8,8 | 9,9 | 10,7 |
| 3-PSE | 94,9 | 93,8 | 92,4 | 86,9 | 82,2 | 86,2 | 85,4 | 75,3 |
| trans-2-PSE | 1,6 | 2,5 | 2,5 | 3,7 | 8,0 | 4,8 | 4,7 | 13,1 |
| cis-2-PSE | - | - | - | 0,4 | - | - | - | 0,9 |

## Patentansprüche

1. Verfahren zur Herstellung von 4-Pentensäureestern durch Isomerisierung, wobei man isomere Pentensäureester bei erhöhter Temperatur mit Zeolithen behandelt und 4-Pentensäureester aus dem Reaktionsgemisch abdestilliert, dadurch gekennzeichnet, daß man Zeolithe des Pentasiltyps verwendet.

2. Verfahren nach Anspruch 1, dadurch gekennzeichnet, daß man Aluminiumsilikatzeolithe verwendet.

3. Verfahren nach Anspruch 1, dadurch gekennzeichnet, daß man Borosilikatzeolithe verwendet.

4. Verfahren nach Anspruch 1, dadurch gekennzeichnet, daß man Eisensilikatzeolithe verwendet.

5. Verfahren nach den Ansprüchen 1 bis 4, dadurch gekennzeichnet, daß die Zeolithe einen Gehalt an Metallen der VIII. Gruppe des Periodensystems haben.

6. Verfahren nach den Ansprüchen 1 bis 5, dadurch gekennzeichnet, daß die Zeolithe des Pentasiltyps einen Gehalt an Palladium, Platin und/oder Rhodium haben.

7. Verfahren nach den Ansprüchen 1 bis 5, dadurch gekennzeichnet, daß die Zeolithe des Pentasiltyps einen Gehalt an Kobalt und/oder Nickel haben.

8. Verfahren nach den Ansprüchen 1 bis 7, dadurch gekennzeichnet, daß die Zeolithe des Pentasiltyps einen Gehalt von 0,1 bis 7,0 Gew.-% an Metallen haben.

9. Verfahren nach den Ansprüchen 1 bis 8, dadurch gekennzeichnet, daß die Zeolithe des Pentasilittyps einen Gehalt an Alkali- und/oder Erdalkaliionen haben.

10. Verfahren nach den Ansprüchen 1 bis 9, dadurch gekennzeichnet, daß die Zeolithe des Pentasiltyps einen Gehalt an seltenen Erdmetallen haben.

## Claims

1. A process for the preparation of a 4-pentenoate by isomerization, in which isomeric pentenoates are treated with a zeolite at elevated temperatures, and the 4-pentenoate is distilled off from the reaction mixture, wherein a zeolite of the pentasil type is used.

2. A process as claimed in claim 1, wherein an aluminosilicate zeolite is used.

3. A process as claimed in claim 1, wherein a borosilicate zeolite is used.

4. A process as claimed in claim 1, wherein an ironsilcate zeolite is used.

5. A process as claimed in any of claims 1 to 4, wherein the zeolite contains a metal of group VIII of the periodic table.

6

6. A process as claimed in any of claims 1 to 5, wherein the zeolite of the pentasil type contains palladium, platinum and/or rhodium.

7. A process as claimed in any of claims 1 to 5, wherein the zeolite of the pentasil type contains cobalt and/or nickel.

8. A process as claimed in any of claims 1 to 7, wherein the zeol ite of the pentasil type contains from 0.1 to 7.0 % by weight of metal.

9. A process as claimed in any of claims 1 to 8, wherein the zeolite of the pentasilite [sic] type contains alkali metal and/or alkaline earth metal ions.

10. A process as claimed in any of claims 1 to 9, wherein the zeolite of the pentasil type contains a rare earth metal.

## Revendications

1. Procédé de préparation d'esters d'acide pentène-4-oïque par isomérisation, en traitant un ester d'acide penténique avec des zéolithes, à température élevée, et en distillant du mélange de réaction l'ester d'acide 4-penténique, caractérisé par le fait que l'on utilise des zéolithes du type pentasile.

2. Procédé selon la revendication 1, caractérisé par le fait qu'on utilise des zéolithes de silicate d'aluminium.

3. Procédé selon la revendication 1, caractérisé par le fait qu'on utilise des zéolithes de silicate de bore.

4. Procédé selon la revendication 1, caractérisé par le fait qu'on utilise des zéolithes de silicate de fer.

5. Procédé selon les revendications 1 à 4, caractérisé par le fait que les zéolithes ont une teneur en métaux du groupe VIII du Système Périodique.

6. Procédé selon les revendications 1 à 5, caractérisé par le fait que les zéolithes du type pentasil ont une teneur en palladium, platine et/ou rhodium.

7. Procédé selon les revendications 1 à 5, caractérisé par le fait que les zéolithes du type pentasil ont une teneur en cobalt et/ou nickel.

8. Procédé selon les revendications 1 à 7, caractérisé par le fait que les zéolithes du type pentasil ont une teneur de 0,1 à 7,0 % en poids en métaux.

9. Procédé selon les revendications 1 à 8, caractérisé par le fait que les zéolithes du type pentasil ont une teneur en ions alcalins et/ou alcalino-terreux.

10. Procédé selon les revendications 1 à 9, caractérisé par le fait que les zéolithes du type pentasil ont une teneur en métaux rares.